(19) **Europäisches Patentamt**

**European Patent Office**

**Office européen des brevets**

(11) Publication number : **0 435 915 B1**

(12) # EUROPEAN PATENT SPECIFICATION

(45) Date of publication of patent specification :
26.05.93 Bulletin 93/21

(51) Int. Cl.⁵ : **A61K 31/71, A61K 47/16**

(21) Application number : **89910641.3**

(22) Date of filing : **19.09.89**

(86) International application number :
**PCT/GB89/01096**

(87) International publication number :
**WO 90/03174 05.04.90 Gazette 90/08**

(54) COMPOSITIONS FOR THE TREATMENT OF LEISHMANIASIS.

(30) Priority : **20.09.88 GB 8822052**

(43) Date of publication of application :
**10.07.91 Bulletin 91/28**

(45) Publication of the grant of the patent :
**26.05.93 Bulletin 93/21**

(84) Designated Contracting States :
**BE CH DE FR GB IT LI LU NL SE**

(56) References cited :
**CHEMICAL ABSTRACTS, vol. 102, no. 4, 28
January 1985, Columbus, OH (US); J.EL-ON et
al., p. 476, no. 32133m***
**CHEMICAL ABSTRACTS, vol. 106, no. 8, 23
February 1987, Columbus, OH (US); W.WOH-
LRAB, p. 367, no. 55812d***

(73) Proprietor : **FARMITALIA CARLO ERBA S.r.L.
Via Carlo Imbonati 24
I-20159 Milano (IT)**

(72) Inventor : **NEAL, Ralph Arthur
Deceased (GB)**
Inventor : **MURPHY, Anthony Gabriel
47 St. Georges Road Palmers Green
London N13 4AT (GB)**

(74) Representative : **Woods, Geoffrey Corlett et al
J.A. KEMP & CO. 14 South Square Gray's Inn
London WC1R 5LX (GB)**

Jouve, 18, rue Saint-Denis, 75001 PARIS

## Description

The present invention relates to compositions for the topical treatment of cutaneous leishmaniasis.

Leishmaniasis, a disease caused by infection with flagellated protozoa of the genus Leishmania, is endemic in many parts of the world. The clinical form of the disease depends upon the species of Leishmania involved and may be visceral, mucocutaneous or cutaneous. Cutaneous leishmaniasis manifests itself in skin lesions and can cause morbidity and mortality as well as giving rise to considerable disfigurement and scarring.

No entirely satisfactory treatment of cutaneous leishmaniasis is currently available; those drugs which have been known for some time for systemically treating leishmaniasis, such as the pentavalent antimonials, have the disadvantages of having variable activity and requiring prolonged parenteral administration which may give rise to toxicity.

In more recent times, the aminoglycoside antibiotic paromomycin, 0-2,6-diamino-2,6-dideoxy-$\beta$-L-idopyranosyl-(1$\rightarrow$3)-0-$\beta$-D-ribofuranosyl-(1$\rightarrow$5)-0-[2-amino-2-deoxy-$\alpha$-D-glucopyranosyl-(1$\rightarrow$4)]-2-deoxystreptamine, has been administered parenterally with beneficial effects in the systemic treatment of both experimental and naturally occurring cutaneous leishmaniasis. However, work in this area has so far been mainly experimental. GB-A-2117237 discloses a synergistic composition for the topical treatment of cutaneous leishmaniasis which comprises paromomycin together with a second compound which itself has topical anti-infective activity. The compounds used as the second component were quaternary ammonium compounds, particularly methylbenzethonium chloride, and dimethyl sulphoxide (DMSO). GB-A-2117237 reports that, whereas paromomycin and the second anti-infective agent used individually were ineffective when applied topically to leishmaniasis lesions, a combination of the two was effective both in the topical treatment of BALB/c mice and in subsequent clinical studies on human patients.

It has now surprisingly been found, in independent experimental studies on the combined paromomycin and quaternary ammonium compound formulations, that local toxicity is associated with the application of a combined paromomycin and quaternary ammonium compound composition to cutaneous leishmaniasis lesions, which is so severe that treatment cannot be continued. In one particular test, the application of a paromomycin ointment comprising 12% methylbenzethonium chloride to lesions in BALB/c mice led to great distress amongst the mice for up to 30 minutes after application and they were observed to become hyperactive using only their front feet and kicking with their hind legs.

We have found that the application of a composition comprising paromomycin in a suitable carrier, in the absence of either a quaternary ammonium compound or DMSO, gave effective results against leishmaniasis lesions in BALB/c mice without causing any local toxicity.

Paromomycin is a large molecule and does not penetrate the skin easily. In practice, it is generally used in the form of an acid addition salt or ester, typically paromomycin sulphate. In general, there are a number of approaches which may be adopted to aid the absorption of an active ingredient through the skin. One way is to emulsify a solution of the active ingredient to give a cream or ointment, for which purpose an emulsifying agent is typically used.

Wool fat (lanolin) is an example of a commonly used emulsifying agent and was initially selected for use with paromomycin. Compositions consisting of paromomycin and wool fat in an inert carrier were formulated with varying amounts of the two ingredients and were tested against lesions caused by <u>Leishmaniasis major</u> in mice. The results achieved seemed promising, but when the compositions were later used in clinical trials they were found to have little effect. This is likely to be due to the difference in skin structure between mice and humans.

An alternative to forming an emulsion as a means of promoting the absorption of an active ingredient through the skin is to formulate the active ingredient with a transdermal penetration enhancing agent. Such an agent was therefore sought for use with paromomycin, it being a requirement that the agent should not exhibit the toxicity of quaternary ammonium compounds. Urea is one of the many available options and when combined with paromomycin in an inert carrier it was found to give a composition having a surprisingly high clinical efficacy. This was particularly unexpected in view of the poor results achieved with wool fat.

Accordingly, the present invention provides a composition suitable for topical administration in the treatment of cutaneous leishmaniasis which consists essentially of a non-toxic carrier, urea and, as sole therapeutic or prophylactic agent, paromomycin or a non-toxic acid addition salt or ester thereof.

Specifically, the composition is free of quaternary ammonium compounds such as methylbenzethonium chloride, which has topical anti-infective activity.

The invention further provides a composition for use in a method of topically treating cutaneous leishmaniasis which consists essentially of a non-toxic carrier, urea and, as sole therapeutic or prophylactic agent, paromomycin or a non-toxic acid addition salt or ester thereof.

In addition, the invention provides the use of paromomycin or a non-toxic acid addition salt or ester thereof

in the preparation of a composition for the topical treatment of cutaneous leishmaniasis, the composition consisting essentially of a non-toxic carrier, urea and, as sole therapeutic or prophylactic agent, paromomycin or a non-toxic acid addition salt thereof.

Accordingly, it is now possible to provide a means of treating cutaneous leishmaniasis which is very effective but which involves none of the local toxicity associated with the prior art paromomycin formulations of GB-A-2117237. It is therefore an important development in the field of topical leishmaniasis treatment.

Hydrocarbon bases such as hard paraffin or white soft paraffin are particularly suitable as the inert carrier, but other carriers or combinations of carriers may be used. Examples include emulsifying bases, non-toxic solvents and water-soluble bases. In particular, in areas where cutaneous leishmaniasis is endemic the ambient temperature is frequently high, for example 40°C or above, and this needs to be taken into account in selecting a carrier that adheres well to the skin. Typically, the carrier or carriers form the balance of the composition and the total proportion by weight of carrier or carriers in the composition is from 99% to 50%, for example 94% to 50%, or 85% to 60%. A proportion of 80% or 75% is particularly suitable.

In addition to ointments and creams, other suitable pharmaceutical presentations for the composition of the invention include lotions and paints. In addition, it is suitable for a medicated dressing to be impregnated with the composition of the invention.

A practical range for the content of paromomycin or salt or ester in the composition is from 1 to 20% by weight, for example 3 to 20% or from 5 to 20%. 8 to 16%, for example 9 to 15% or 10 to 14%, is particularly suitable. As mentioned above, the non-toxic salt of paromomycin is typically the sulphate. The ester is typically an alkyl ester, for example a $C_1$-$C_4$ alkyl ester.

The paromomycin may be incorporated in the composition, for example when it is an ointment, as a very fine powder (British Pharmacopoeia). The particle size is generally 125 μm or less and is a parameter that may influence the degree of transdermal penetration of the drug. Accordingly, micronization of the paromomycin prior to its incorporation into the ointment should lead to a reduction in the amount of paromomycin required for an effective result.

The urea is generally incorporated into the composition as a fine powder, typically having a particle size of 125 μm or less. It is suitably used in an amount of from 5 to 30%, for example from 8 to 25% or 10 to 20% by weight. A, amount of 10% by weight is particularly suitable.

The composition of the invention is prepared by mixing the paromomycin or a non-toxic acid addition salt or ester thereof with the urea and the carrier. Standard methods are suitably used in preparing pharmaceutical presentations. For example, when the composition is presented in the form of an ointment, this is suitably prepared by dispersing a fine powder of paromomycin or a non-toxic salt or ester thereof with urea in an ointment base at room temperature. When the composition takes the form of a cream, this is suitably prepared by dissolving the urea and the paromomycin or a non-toxic salt or ester thereof in water and mixing the resulting solution with a cream base, at a temperature of from 50°C to 70°C.

A suitable method of treatment of the human or animal body using the composition of the present invention comprises applying to skin infected with protozoa of the genus Leishmania a therapeutically effective amount of a composition consisting essentially of a non-toxic carrier, urea and, as sole therapeutic or prophylactic agent, paromomycin, or a non-toxic acid addition salt or ester thereof, by a topical route.

The composition is generally applied to each lesion several times per day, suitably twice per day. Typically the composition is applied from a tube fitted with a nozzle having a 5mm internal diameter. The amount of paromomycin and urea contained in a 1cm length of ointment extruded from such a tube was determined as follows.

Polystyrene weighing boats were numbered and a 1 cm length was marked with a ballpoint pen. Each boat was tared and ointment containing 15% paromomycin sulphate and 10% urea in a white soft paraffin base was squeezed through a nozzle having a 5mm internal diameter as described above. The ointment was square off on the nozzle before extrusion and cut at the 1cm mark with a scalpel. The boats were reweighed to give the mass of ointment. The results obtained for fifteen measurements are tabulated below:

| Boat No. | Mass of ointment (mg) | | Boat No. | Mass of ointment (mg) |
|---|---|---|---|---|
| 1 | 194 | | 9 | 209 |
| 2 | 198 | | 10 | 183 |
| 3 | 187 | | 11 | 188 |
| 4 | 164 | | 12 | 212 |
| 5 | 209 | | 13 | 177 |
| 6 | 207 | | 14 | 173 |
| 7 | 190 | | 15 | 196 |
| 8 | 190 | | | |

The mean mass of ointment = 191.8 ± 3.5 mg

On this basis, the amount of paromomycin applied to a lesion 1cm in diameter (e.g. on a mouse) is about 29mg and the amount of urea contained in the dose is about 19mg. Lesions in humans usually have a larger diameter, however, and may be, for example, 3cm across. The ointment is then conveniently applied by a "cross" technique, whereby two lengths of ointment are squeezed across the lesion at right angles to one another and then spread out. This means effectively that six 1cm doses of ointment are applied to a 3cm diameter lesion, giving about 173mg of paromomycin and about 115mg of urea per treatment.

The composition may also be applied by any other suitable means and is usually applied to give a dose of from 2mg to 60mg, for example from 10mg to 30mg, of paromomycin per 1cm of lesion per application.

The following Examples further illustrate the present invention.

EXAMPLE 1: Composition preparation

The following two compositions were prepared as ointments, by dispersing paromomycin sulphate powder and urea in white soft paraffin (WSP) at room temperature. The paromomycin sulphate particle size was 125 μm and the urea particle size was less than 125 μm. All percentages are by weight.

A: Paromomycin sulphate 15%
   Urea 10%
   WSP balance
B: Paromomycin sulphate 10%
   Urea 10%
   WSP balance

Comparative Example 1

The following compositions were prepared; with paromomycin particle size of 125 microns.

C: Paromomycin sulphate 15%
   Wool fat 35%
   WSP 25%
   Water 25%
D: Paromomycin sulphate 10%
   WSP balance
E: Paromomycin sulphate 10%
   Wool fat 42.8%
   Water 16.7%
   WSP 30.6%
F: Paromomycin sulphate 5%
   Wool fat 50.5%
   Water 8.5%
   WSP 36%
G: Paromomycin sulphate 15%
   WSP balance
H: Paromomycin sulphate 15%
   Methylbenzethonium chloride 12%
   WSP balance

4

I:      Wool fat 50%
        WSP 25%
        Water 25%

Compositions A, B, D and H were ointments, prepared by dispersing paromomycin sulphate powder as very fine particles in WSP at room temperature.

Compositions C, E and F were creams, prepared by dissolving paromomycin sulphate in water and mixing this solution with WSP and wool fat at 60°C. The cream was then stirred until cool.

Composition I, a cream, was prepared by mixing together wool fat and WSP at 60°C and stirring until cool.

Composition H is a formulation disclosed in GB-A-2117237.

EXAMPLE 2: In Vivo tests in mice

The compositions prepared according to Example 1, and Comparative Example 1 were used as follows.

For a cutaneous lesion of 10mm diameter, a 10 mm length of composition was squeezed from a tube having a nozzle of 5 mm internal diameter. The mean mass of composition applied was 203 ± 4.6 mg; since the proportion of paromomycin used was from 5% to 15% the average topical dose of paromomycin was therefore from approximately 10 mg to 30 mg per application.

The experimental system was BALB/c mice inoculated in the rump with $1 \times 10^7$ promastigotes of a Saudi Arabian strain of L. major JISHA 118 clone 6. After the lesions had developed, the topical treatment was given twice daily for 10 days. The effect of treatment was monitored by weekly measurements of the diameter of the lesion for up to 4 months. The lesions were mostly ulcerations but closed nodules were present at the start of the experiments in some animals.

The results are listed in Tables 1 and 2. Apart from the composition containing the quaternary ammonium compound, composition H, there was little change in lesion size during the 10 day treatment. However, the composition A comprising urea was relatively successful. Thereafter the lesion lost the raised edges, the ulceration filled in by granulation tissue and epithelium covered the site. Hair slowly grew back over the site. The effect was slow, the maximum effect being first observed 3-4 weeks after completion of treatment.

Mice treated with the composition containing the quaternary ammonium compound, methylbenzethonium chloride, were very distressed for about 30 minutes after application. They were hyperactive only using their front feet, had hunched backs and kicked with their hind legs. By the seventh day of treatment, the sores were large, necrotic, black in colour and the surrounding skin had lost hair, with erythema and dead and sloughing skin. However, after application of the composition had been completed, the lesions did slowly heal. The scar sites were larger and more inflamed than with other treatments.

<u>TABLE 1</u>

Topical application of formulations comprising 15%
paromomycin to BALB/c mice with <u>L. major</u> lesions

| Composition | Treatment | Mean lesion diameter mm | Mice without lesions | |
|---|---|---|---|---|
| | | Week 4 | Week 4 | Month 3 |
| - | Untreated | 15.5±0.5 | 0/7 | 0/7 |
| G | White soft paraffin | 0.1±0.5 | 6/7 | 0/7 |
| A | Urea 10% in white soft paraffin | 1.3±1.0 | 5/7 | 1/7 |
| C | White soft paraffin, water hydrous wool fat | 1.2±1.0 | 6/7 | 2/7 |
| H | Formulation of GB-A-2117237 | O | 5/7 | 1/7 |

## TABLE 2

Topical application of formulations comprising differing

amounts of paromomycin to BALB/c mice with <u>L. major</u> lesions

| Composition | Paromomycin (%) | Mean lesion diameter mm week 3 | Mice without lesions Week 3 | Month 3 |
|---|---|---|---|---|
| | — | 12.6±1.1 | 0/7 | – |
| I | None | 11.9±1.2 | 0/7 | – |
| E | 15% | 0 | 7/7 | 7/7 |
| D | 10% | 0 | 7/7 | 7/7 |
| F | 5% | 1.0±0.9 | 1/7 | 0/7 |
| G | 15% | 0.4±0.4 | 1/6 | 6/6 |
| A | 15% | 0 | 6/6 | 6/6 |

EXAMPLE 3: Clinical Tests

Patients suffering from cutaneous lesions due to L.major, L.infantum or L.tropica were treated topically with composition A of Example 1 and compositions C and H of Comparative Example 1. Two lengths at right angles to each other were placed across each lesion from a tube having a 5 mm internal diameter as described in Example 5. The ointment was then spread over the lesion in a layer of uniform thickness.

The results shown in Table 3 illustrate that although the use of methylbenzethonium chloride as adjuvant with paromomycin gives therapeutic results approaching those achieved with the use of urea, the severe local toxicity associated with it renders the composition unacceptable for pharmaceutical use. Urea is therefore seen to be the most effective transdermal enhancing agent.

TABLE 3

| Composition | (Adjuvant with paromomycin) | No. of patients treated | No. of patients treated | Failure | No. of patients where toxicity observed |
|---|---|---|---|---|---|
| A | Urea | 7 | 6 | 1* | 1** |
| C | Wool fat | 3 | 0 | 3 | 0 |
| H | Methylbenzeth- onium chloride | 12 | 10 | 2 | 10 |

Footnotes

\* : Parasite cure but failure of lesion to heal due to ischaemic leg

\*\* : Not toxic to this patient on repeat application

**Claims**

1. A composition suitable for topical application for the treatment of cutaneous leishmaniasis which consists essentially of a non-toxic carrier, urea and, as sole therapeutic or prophylactic agent, paromomycin or a non-toxic acid additional salt or ester thereof.

2. A composition according to claim 1 wherein the non-toxic acid addition salt is paromomycin sulphate.

3. A composition according to claim 1 wherein the carrier is white soft paraffin.

4. A composition according to claim 1 comprising paromomycin or a non-toxic acid addition salt or ester thereof in an amount of from 8 to 16% by weight.

5. A composition according to claim 1 comprising urea in an amount of from 5 to 30% by weight.

6. A composition according to claim 1 wherein the paromomycin, its salt or ester is incorporated in micronised form.

7. A process for preparing a composition as claimed in claim 1 which comprises mixing the paromomycin or non-toxic acid addition salt or ester thereof with the urea and the carrier.

8. A process according to claim 7 which comprises dispersing a fine powder of the paromomycin or salt or ester thereof with a fine powder of urea in an ointment base, or dissolving the paromomycin or salt or ester thereof and the urea in water and mixing the resulting solution with a cream base.

9. Use of paromomycin, or a non-toxic acid addition salt or ester thereof, in the preparation of a composition for the topical treatment of cutaneous leishmaniasis, the composition consisting essentially of a non-toxic carrier, urea and as sole therapeutic or prophylactic agent, paromomycin or a non-toxic acid addition salt or ester thereof.

**Patentansprüche**

1. Zusammensetzung, welche geeignet ist zur topischen Applikation zur Behandlung von Haut-Leishmaniose, bestehend im wesentlichen aus einem nicht-toxischen Träger, Harnstoff und als einzigem therapeutischen oder prophylaktischen Mittel Paromomycin oder einem seiner nicht-toxischen Säureadditionssalze oder Ester.

2. Zusammensetzung gemäss Anspruch 1, worin das nicht-toxische Säureadditionssalz Paromomycinsulfat ist.

3. Zusammensetzung gemäss Anspruch 1, worin der Träger weisses Weichparaffin ist.

4. Zusammensetzung gemäss Anspruch 1, umfassend Paromomycin oder eines seiner nicht-toxischen Säureadditionssalze oder Ester in einer Menge von 8 bis 16 Gew.%.

5. Zusammensetzung gemäss Anspruch 1, umfassend Harnstoff in einer Menge von 5 bis 30 Gew.%.

6. Zusammensetzung gemäss Anspruch 1, worin das Paromomycin oder sein Salz oder Ester in mikrozerkleinerter Form vorliegt.

7. Verfahren zur Herstellung einer Zusammensetzung gemäss Anspruch 1, umfassend das Mischen des Paromomycins oder dessen nicht-toxischen Additionssalzes oder Esters mit dem Harnstoff und dem Träger.

8. Verfahren gemäss Anspruch 7, umfassend das Dispergieren eines feinen Pulvers von Paromomycin oder dessen Salz oder Ester mit einem feinen Harnstoffpulver in einer Salbengrundlage, oder das Auflösen von Paromomycin oder dessen Salz oder Ester und von Harnstoff in Wasser, und Mischen der resultierenden Lösung mit einer Creme-Grundlage.

9. Verwendung von Paromomycin oder einem seiner nicht-toxischen Säureadditionssalze oder Ester zur Herstellung einer Zusammensetzung für die topische Behandlung von Haut-Leishmaniose, wobei die Zusammensetzung im wesentlichen aus einem nichttoxischen Träger, Harnstoff und als einzigem therapeutischen oder prophylaktischen Mittel Paromomycin oder einem seiner nicht-toxischen Säureadditionssalze oder Ester besteht.

**Revendications**

1. Composition appropriée pour une application topique en vue du traitement de la leishmaniose cutanée, qui est essentiellement constituée par un support non-toxique, d'urée et, en tant qu'unique agent thérapeutique ou prophylactique, de la paromomycine ou un sel non-toxique d'addition avec un acide ou un ester non-toxique de celle-ci.

2. Composition selon la revendication 1, dans laquelle le sel non-toxique d'addition avec un acide est le sulfate de paromomycine.

3. Composition selon la revendication 1, dans laquelle le support est de la paraffine molle blanche.

4. Composition selon la revendication 1, comprenant de la paromomycine ou un sel non-toxique d'addition avec un acide ou un ester non-toxique de celle-ci, dans une quantité de 8 à 16% en poids.

5. Composition selon la revendication 1, comprenant de l'urée dans une quantité de 5 à 30% en poids.

6. Composition selon la revendication 1, dans laquelle la paromomycine, son sel ou son ester, est incorporé sous une forme micronisée.

7. Procédé de préparation d'une composition telle que définie à la revendication 1, qui comprend le mélange de la paromomycine ou d'un sel non-toxique d'addition avec un acide ou d'un ester non-toxique de celle-ci, avec l'urée et le support.

8. Procédé selon la revendication 7, qui comprend la dispersion d'une poudre fine de la paromomycine ou d'un sel ou ester de celle-ci avec une poudre fine d'urée dans une base pour pommade, ou bien la dissolution dans l'eau de !a paromomycine ou d'un sel ou ester de celle-ci et de l'urée, et le mélange de la solution résultante avec une base pour crème.

9. Utilisation de la paromomycine, ou d'un sel non-toxique d'addition avec un acide ou d'un ester non-toxique de celle-ci, dans la préparation d'une composition pour le traitement topique de la leishmaniose cutanée, la composition étant essentiellement constituée par un support non-toxique, d'urée et, en tant qu'unique agent thérapeutique ou prophylactique, la paromomycine ou un sel non-toxique d'addition avec un acide ou un ester non-toxique de celle-ci.